# EUROPÄISCHE PATENTSCHRIFT

(11) **EP 1 972 321 B1**
(45) Veröffentlichungstag und Bekanntmachung des Hinweises auf die Patenterteilung: **24.12.2014**
(21) Anmeldenummer: 07104431.7
(22) Anmeldetag: 19.03.2007
(51) Int. Cl.: A61K 6/04

(54) **Verfahren zur Herstellung eines Zahnersatzes sowie Zahnersatz**
Method for manufacturing a dental prosthesis and a dental prosthesis
Procédé destiné à la fabrication d'une prothèse dentaire, tout comme prothèse dentaire

(43) Veröffentlichungstag der Anmeldung: 24.09.2008
(73) Patentinhaber: Weisensel, Lars, 90475 Nürnberg (DE); Bauer, Jochen, 78239 Rielasingen (DE)
(72) Erfinder: Bauer, Jochen, Dr., 78239 Rielasingen-Worblingen (DE); Klaus, Angela, 63456 Hanau (DE); Steinke, Rudi, 63457 Hanau (DE); Weisensel, Lars, 63867 Johannesberg (DE)
(74) Vertreter: Hoefer & Partner

(56) Entgegenhaltungen:
- EP-A- 1 696 044
- EP-A1- 1 568 472
- WO-A-2004/042098
- DE-A1- 19 815 091
- DE-C1- 3 319 457
- DE-C1- 19 649 865

## Beschreibung

Die Erfindung bezieht sich auf ein Verfahren zur Herstellung eines Zahnersatzes sowie eine Kobalt-Chrom-Legierung zur Verwendung dieser.

Aus der DE-C-33 19 457 ist eine Kobalt-Chrom-Legierung bekannt, die als Aufbrennlegierung für einen fest sitzenden oder herausnehmbaren Zahnersatz bestimmt ist und hinsichtlich Aufschmelz- und Gießverhalten optimiert ist. Dabei weist die Legierung nach der für das Verblenden mit Keramik erforderlichen Wärmebehandlung Härten von weniger als 300 (HV 5) auf.

Um kostengünstig hochpräzise einen Zahnersatz beliebiger Geometrie herzustellen, ist es bekannt, Zahnersatz mit dem Laserschmelz- oder Sinterverfahren zu produzieren. Dabei gelangt ein Verfahren zum Einsatz, das prinzipiell aus der DE-C-196 49 865 bekannt ist. Zur Herstellung wird dabei pulverförmiges Material nacheinander in Schichten auf einen Träger aufgebracht, wobei jede Schicht vor dem Aufbringen der nächstfolgenden Schicht mit einem fokussierten Laserstrahl derart erwärmt wird, das ein Fixieren auf der darunter liegenden Pulverschicht erfolgt.

Als Aufbrennlegierungen, die für ein entsprechendes Verfahren verwendet werden können, werden Kobalt-Chrom-Legierungen benutzt. Dabei müssen die nach dem Lasersinter- bzw. -schmelzverfahren hergestellten Gerüste zumindest nach der Herstellung und vor dem Verblenden einer Temperaturbehandlung unterzogen werden, um zu versuchen, die Verzugsneigung zu reduzieren.

Nachteilig bei der Herstellung von Zahnersatz durch Lasersinterung bzw. Laserschmelzen ist jedoch, dass die verwendeten Legierungen, insbesondere Kobalt-Chrom-Werkstoffe nach dem Lasersintern bzw. -schmelzen eine extrem hohe Härte HV > 400 aufweisen. Dabei ist des Weiteren festzustellen, dass während der keramischen Verblendung eine weitere Versprödung auftritt. Die starke Sprödigkeit führt dabei zu einer ausgeprägten Bruchneigung. Des Weiteren haben Untersuchungen ergeben, dass zahntechnische Objekte, die aus Kobalt-Chrom-Werkstoffen durch Lasersintern bzw. - schmelzen hergestellt worden sind, eine hohe Verzugsneigung zeigen.

Aus der EP-A-1 568 472 ist ein Verfahren zur Herstellung von Dentalprodukten durch Freiform-Sintern bzw. -Schmelzen bekannt, wobei Schichten nacheinander mehrfach bestrahlt werden, um diese zum einen miteinander zu verschmelzen und zum anderen eine gewünschte Form herzustellen.

Die EP-A-1696 044 hat eine aufbrennfähige Kobalt-Chrom-Gießlegierung zur Herstellung keramisch verblendeter Dentalrestaurationen zum Gegenstand.

Aus der DE-A-198 15 091 ist eine Legierung für Dentalgussteile bekannt, bei der der Wolframanteil 0,5 Gew.% beträgt. Ferner ist zwingend Stickstoff enthalten.

Eine im Wesentlichen eisenfreie Metallgießlegierung ist Gegenstand der WO-A-2004/042098.

Der vorliegenden Erfindung liegt die Aufgabe zugrunde, einen Werkstoff zur Verfügung zu stellen, um eine Optimierung einer zahntechnischen Produktqualität zu erreichen. Dabei soll das Verarbeitungsverhalten des zum Einsatz gelangenden Werkstoffs im Vergleich zum Stand der Technik verbessert werden. Die Verzugsneigung soll minimiert und eine Versprödung während der keramischen Verblendung derart reduziert werden, dass eine Bruchneigung minimiert wird.

Zur Lösung der Aufgabe schlägt die Erfindung ein Verfahren zur Herstellung eines Zahnersatzes durch schichtweises Aufbauen aus Pulver einer Kobalt-Chrom-Legierung vor, bei dem nacheinander mehrere Pulverschichten aufgebracht und jede Pulverschicht vor Aufbringen der nächstfolgenden Pulverschicht mit einem fokussierten Laserstrahl in einem vorgegebenen Bereich, der einem ausgewähltem Querschnittsbereich des herzustellenden Zahnersatzes entspricht, auf eine vorgegebene Temperatur derart erhitzt wird, dass die Pulverschicht an der darunter liegenden Pulverschicht durch Aufschmelzen der Pulverschicht und/oder Sintern fixiert wird, und zeichnet sich dadurch aus, dass als Kobalt-Chrom-Legierung verwendet wird eine solche mit
43 - 68 Gew.-% Kobalt,
12 - 30 Gew.-% Chrom,
8 - 25 Gew.-% Wolfram,
0 - 13 Gew.-% Eisen,
0 - 30 Gew.-% Mangan,
0 - 10 Gew.-% Molybdän,
0 - 5 Gew.-% zumindest eines der Elemente
Aluminium, Tantal, Rhenium, Titan
und weniger als 0,1 Gew.-% Kohlenstoff.

Dabei ist insbesondere vorgesehen, dass der Zahnersatz nach dessen Herstellung durch das Lasersinter- und/oder -schmelzverfahren ohne vorhergehende Wärmebehandlung mit einer Verblendung aus Keramik oder Kunststoff versehen wird.

Überraschenderweise hat sich gezeigt, dass bei der Verwendung einer entsprechenden Kobalt-Chrom-Legierung der durch Lasersinter- bzw. -schmelzverfahren fertig gestellte Zahnersatz eine Härte aufweist, die für Gusslegierungen üblich ist, jeweils vor der Verblendung. Nachteile bezüglich einer etwaigen Verzugsneigung sind nicht gegeben.

Des Weiteren konnte festgestellt werden, dass mit der erfindungsgemäß verwendeten Kobalt-Chrom-Legierung ohne Temperaturbehandlung vor oder nach dem Sinterprozess verzugsfreie Gerüste mit einer gusstypischen Härte ausreichender Duktilität auch nach dem Verblenden zur Verfügung stehen.

Insbesondere ist vorgesehen, dass der hergestellte Zahnersatz ohne vorherige Temperung verblendet wird.

Auch wird die Herstellung des Zahnersatzes durchgeführt, ohne dass während des Sinter- bzw. Schmelzprozesses eine zusätzliche Wärmebehandlung erfolgt, wie dies z.B. beim Stand der Technik durch Aufheizen der Prozesskammer der Fall ist.

Insbesondere ist vorgesehen, dass die Kobalt-Chrom-Legierung zusätzlich 0 - 0,2 Gew.-% zumindest eines der Elemente Bor, Yttrium, 0 - 2 Gew.-% zumindest eines der Elemente Vanadium, Silizium, Kupfer, Zink, Niob, 0 - 10 Gew.-% Gallium, 0 - 5 Gew.-% Germanium und 0 - 1 Gew.-% zumindest eines der Elemente Cer, Lanthan enthält. Dabei sollte die Legierung nicht mehr als 50 Gew.-% Chrom, Wolfram und Rhenium enthalten. Des Weiteren zeichnet sich die Erfindung dadurch aus, dass das Verhältnis von Chrom : Wolfram + Rhenium zwischen ca. 2:3 und ca. 2:1 bzw. zwischen ca. 2:3 und ca. 3:2 liegt.

In Ausgestaltung sieht die Erfindung vor, dass der Gehalt von Chrom + Wolfram mindestens 30 Gew.-% und höchstens 40 Gew.-% ist und das Verhältnis von Chrom : Wolfram zwischen ca. 3:4 und ca. 4:3 liegt.

Insbesondere kann die Legierung 50 - 65 Gew.-% Kobalt, 15 - 22 Gew.-% Chrom, 15 - 22 Gew.-% Wolfram, 4 - 11 Gew.-% Eisen und 1 - 3 Gew.-% Aluminium enthalten.

Die Erfindung zeichnet sich jedoch auch aus durch die Verwendung einer Kobalt-Chrom-Legierung mit
43 - 68 Gew.-% Kobalt,
12 - 30 Gew.-% Chrom,
8 - 25 Gew.-% Wolfram,
0 - 13 Gew.-% Eisen,
0 - 30 Gew.-% Mangan,
0 - 10 Gew.-% Molybdän,
0-5 Gew.-% zumindest eines der Elemente
Aluminium, Tantal, Rhenium, Titan
und weniger als 0,1 Gew.-% Kohlenstoff
zur Herstellung von Zahnersatz mittels Lasersinter und/oder -schmelzverfahren. Insbesondere ist vorgesehen dass die Kobalt-Chrom-Legierung zusätzlich 0 - 0,2 Gew.-% zumindest eines der Elemente Bor, Yttrium, 0 - 2 Gew.-% zumindest eines der Elemente Vanadium, Silizium, Kupfer, Zink, Niob, 0 - 10 Gew.-% Gallium, 0 - 5 Gew.-% Germanium und 0 - 1 Gew.-% zumindest eines der Elemente Cer, Lanthan enthält. Dabei sollte die Legierung nicht mehr als 50 Gew.-% Chrom, Wolfram und Rhenium enthalten. Des Weiteren zeichnet sich die Erfindung dadurch aus, dass das Verhältnis von Chrom : Wolfram + Rhenium zwischen ca. 2:3 und ca. 2:1 bzw. zwischen ca. 2:3 und ca. 3:2 liegt.

In Ausgestaltung sieht die Erfindung vor, dass der Gehalt von Chrom + Wolfram mindestens 30 Gew.-% und höchstens 40 Gew.-% ist und das Verhältnis von Chrom : Wolf ram zwischen ca. 3:4 und ca. 4:3 liegt.

Insbesondere kann die Legierung 50 - 65 Gew.-% Kobalt, 15 - 22 Gew.-% Chrom, 15 - 22 Gew.-% Wolfram, 4 - 11 Gew.-% Eisen und 1 - 3 Gew.-% Aluminium enthalten.

Die Erfindung ist auch gekennzeichnet durch einen aus einer Kobalt-Chrom-Legierung bestehenden durch Lasersinter- und/oder -schmelzverfahren hergestellten Zahnersatz, der vor dem Verblenden eine Härte HV (HV10) ≤ 350 aufweist.

Überraschenderweise hat sich des Weiteren herausgestellt, dass die Härte eines erfindungsgemäß hergestellten Zahnersatzes wie Gerüstes zunimmt, sofern eine Verblendung mit Keramik erfolgt. Die Härte HV10 beträgt mehr als 400. Auch nehmen die Zugfestigkeit und Streckgrenze zu.

## Patentansprüche

1. Verfahren zur Herstellung eines Zahnersatzes durch schichtweises Aufbauen aus Pulver einer Kobalt-Chrom-Legierung, bei dem nacheinander mehrere Pulverschichten übereinander aufgebracht und jede Pulverschicht vor dem Aufbringen der nächstfolgenden Pulverschicht mit einem fokussierten Laserstrahl in einem vorgegebenen Bereich, der einem ausgewählten Querschnittsbereich des herzustellenden Zahnersatzes entspricht, auf eine vorgegebene Temperatur derart erhitzt wird, dass die Pulverschicht an der darunter liegenden Pulverschicht durch Aufschmelzen der Pulverschicht und/oder Sintern fixiert wird,
**dadurch gekennzeichnet,**
**dass** als Kobalt-Chrom-Legierung verwendet wird eine solche bestehend aus
43 - 68 Gew.-% Kobalt,
12 - 30 Gew.-% Chrom,
8 - 25 Gew.-% Wolfram,
0-13 Gew.-% Eisen,
0 - 30 Gew.-% Mangan,
0 - 10 Gew.-% Molybdän,
0-5 Gew.-% zumindest eines der Elemente
Aluminium, Tantal, Rhenium, Titan und weniger als 0,1 Gew.-% Kohlenstoff.

2. Verfahren nach Anspruch 1,
**dadurch gekennzeichnet,**
**dass** die Kobalt-Chrom-Legierung zusätzlich 0 - 0,2 Gew.-% zumindest eines der Elemente Bor, Yttrium, 0 - 2 Gew.-% zumindest eines der Elemente Vanadium, Silizium, Kupfer, Zink, Niob, 0 - 10 Gew.-% Gallium, 0 - 5 Gew.-% Germanium und 0 - 1 Gew.-% zumindest eines der Elemente Cer, Lanthan enthält.

3. Verfahren nach Anspruch 2,
**dadurch gekennzeichnet,**
**dass** die Legierung nicht mehr als 50 Gew.-% Chrom, Wolfram und Rhenium enthält.

4. Verfahren nach zumindest einem der vorhergehenden Ansprüche,
**dadurch gekennzeichnet,**
**dass** das Verhältnis Chrom : Wolfram + Rhenium zwischen ca. 2:3 und ca. 2:1 bzw. zwischen ca. 2:3 und ca. 3:2 liegt.

5. Verfahren nach zumindest einem der vorhergehenden Ansprüche,
**dadurch gekennzeichnet,**
**dass** der Gehalt von Chrom + Wolfram mindestens 30 Gew.-% und höchstens 50 Gew.-% ist und das Verhältnis von Chrom : Wolfram zwischen ca. 3:4 und ca. 4:3 liegt.

6. Verfahren nach zumindest einem der vorhergehenden Ansprüche,
**dadurch gekennzeichnet,**
**dass** die Legierung 50 - 65 Gew.-% Kobalt, 15 - 22 Gew.-% Chrom, 15 - 22 Gew.-% Wolfram, 4 - 11 Gew.-% Eisen und 1 - 3 Gew.-% Aluminium enthält.

7. Verfahren nach zumindest einem der vorhergehenden Ansprüche,
**dadurch gekennzeichnet,**
**dass** der hergestellte Zahnersatz ohne vorherige Temperung verblendet wird.

8. Verwendung einer Kobalt-Chroni-Legierung, bestehend aus 43 - 68 Gew.-% Kobalt,
12 - 30 Gew.-% Chrom,
8 - 25 Gew.-% Wolfram,
0 - 13 Gew.-% Eisen,
0 - 30 Gew.-% Mangan,
0 - 10 Gew.-% Molybdän,
0 - 5 Gew.-% zumindest eines der Elemente
Aluminium, Tantal, Rhenium, Titan und weniger als 0,1 Gew.-% Kohlenstoff zur Herstellung von Zahnersatz mittels Laserschmelz- und/oder -sinterverfahren.

9. Verwendung der Kobalt-Chrom-Legierung nach Anspruch 8,
die zusätzlich enthält
0 - 0,2 Gew.-% zumindest eines der Elemente Bor, Yttrium, 0 - 2 Gew.-% zumindest eines der Elemente Vanadium, Silizium, Kupfer, Zink, Niob, 0 - 10 Gew.-% Gallium, 0 - 5 Gew.-% Germanium und 0 - 1 Gew.-% zumindest eines der Elemente Cer, Lanthan.

10. Verwendung der Kobalt-Chrom-Legierung nach Anspruch 9 die
nicht mehr als 50 Gew.-% Chrom, Wolfram und Rhenium enthält.

11. Verwendung der Kobalt-Chrom-Legierung nach zumindest einem der Ansprüche 8 - 10,
die ein Verhältnis von Chrom : Wolfram + Rhenium zwischen ca. 2:3 und ca. 2:1 bzw. zwischen ca. 2:3 und ca. 3:2 aufweist.

12. Verwendung der Kobalt-Chrom-Legierung nach zumindest einem der Ansprüche 8-11,
bei der der Gehalt von Chrom + Wolfram mindestens 30 Gew.-% und höchstens 50 Gew.-% ist und das Verhältnis von Chrom : Wolfram zwischen ca. 3:4 und ca. 4:3 liegt.

13. Verwendung der Kobalt-Chrom-Legierung nach zumindest einem der Ansprüche 8-12,
die 50 - 65 Gew.-% Kobalt, 15 - 22 Gew.-% Chrom, 15 - 22 Gew.-% Wolfram, 3 - 11 Gew.-% Eisen und 1 - 3 Gew.-% Aluminium enthält.

14. Aus einer Kobalt-Chrom-Legierung nach zumindest einem der vorhergehenden Ansprüche durch Laserschmelz- und/oder Sinterverfahren hergestellter Zahnersatz ohne Verblendung einer Härte HV (HV10) ≤ 350.

## Claims

1. A method for the manufacture of a dental prosthesis by layer-by-layer build-up from powder of a chromium-cobalt alloy, in which several powder layers are successively applied on top of each other and each powder layer, prior to the application of the subsequent powder layer, is heated in a selected area, corresponding to a selected cross-sectional area of the dental prosthesis to be manufactured, by a focused laser beam to a predetermined temperature in such a manner that the powder layer is attached to the powder layer below by melting and/or sintering of the powder layer,
**characterized in**
**that** the employed cobalt-chromium alloy is one consisting of
43-68% by weight cobalt,
12-30% by weight chromium,
8-25% by weight tungsten,
0-13% by weight iron,
0-30% by weight manganese,
0-10% by weight molybdenum,
0-5% by weight of at least one of the elements
aluminum, tantalum, rhenium, titanium and less than 0.1% by weight carbon.

2. The method of claim 1, **characterized in that** the cobalt-chromium alloy additionally contains 0-0.2% by weight of at least one of the elements boron, yttrium, 0-2% by weight of at least one of the elements vanadium, silicon, copper, zinc, niobium, 0-10% by weight gallium, 0-5% by weight germanium, and 0-1% by weight of at least one of the elements cerium, lanthanum.

3. The method of claim 2, **characterized in that** the alloy does not contain more than 50% by weight of chromium, tungsten, and rhenium.

4. The method of any of the preceding claims, **characterized in that** the chromium:tungsten+rhenium ratio ranges between approximately 2:3 and approximately 2:1 or rather between approximately 2:3 and approximately 3:2.

5. The method of any of the preceding claims, **characterized in that** the content of chromium+tungsten is at least 30% by weight and at most 50% by weight, and that the chromium:tungsten ratio is between approximately 3:4 and approximately 4:3.

6. The method of any of the preceding claims, **characterized in that** the alloy contains 50-65% by weight cobalt, 15-22% by weight chromium, 15-22% by weight tungsten, 4-11% by weight iron, and 1-3% by weight aluminum.

7. The method of any of the preceding claims, **characterized in that** the manufactured dental prosthesis is veneered without prior annealing.

8. Use of a cobalt-chromium alloy consisting of
43-68% by weight cobalt,
12-30% by weight chromium,
8-25% by weight tungsten,
0-13% by weight iron,
0-30% by weight manganese,
0-10% by weight molybdenum,
0-5% by weight of at least one of the elements
aluminum, tantalum, rhenium, titanium and less than 0.1% by weight carbon in the manufacture of a dental prosthesis by laser melting and/or sintering.

9. The use of the cobalt-chromium alloy of claim 8, the cobalt-chromium alloy additionally containing 0-0.2% by weight of at least one of the elements boron, yttrium, 0-2% by weight of at least one of the elements vanadium, silicon, copper, zinc, niobium, 0-10% by weight gallium, 0-5% by weight germanium, and 0-1% by weight of at least one of the elements cerium, lanthanum.

10. The use of the cobalt-chromium alloy of claim 9, the cobalt-chromium alloy containing not more than 50% by weight of chromium, tungsten, and rhenium.

11. The use of the cobalt-chromium alloy of any of claims 8 to 10, in which the chromium:tungsten+rhenium ratio is between approximately 2:3 and approximately 2:1 or rather between approximately 2:3 and approximately 3:2.

12. The use of the cobalt-chromium alloy of any of claims 8 to 11, in which the content of chromium+tungsten is at least 30% by weight and at most 50% by weight and the chromium:tungsten ratio is between approximately 3:4 and approximately 4:3.

13. The use of the cobalt-chromium alloy of any of claims 8 to 12, the cobalt-chromium alloy containing 50-65% by weight cobalt, 15-22% by weight chromium, 15-22% by weight tungsten, 3-11% by weight iron, and 1-3% by weight aluminum.

14. A dental prosthesis produced from a cobalt-chromium alloy by means of a laser melting and/or laser sintering process, which without veneering exhibits a hardness (HV10)≤350.

## Revendications

1. Procédé destiné à la fabrication d'une prothèse dentaire par assemblage de couches de poudre d'un alliage de cobalt-chrome, par lequel plusieurs couches de poudre sont appliquées l'une après l'autre et l'une au-dessus de l'autre, et chaque couche de poudre avant l'application de la couche suivante est chauffée à une température prédéterminée avec un rayon laser focalisé dans une zone prédeterminée correspondant à une section transversale sélectionée de la prothèse dentaire à fabriquer, de façon que la couche de poudre soit fixée à la couche de poudre sous-jacente par refusion de la couche de poudre et/ou par frittage,
**caractérisé en ce que**
comme alliage cobalt-chrome est utilize, lequel se compose de
43 - 68 % en poids de cobalt
12 - 30 % en poids de chrome
8 - 25 % en poids de tungstène
0 -13 % en poids de fer
0 - 30 % en poids de manganèse
0 -10 % en poids de molybdène
0 - 5 % en poids d'au moins un des éléments
aluminium, tantale, rhénium, titane et de moins que 0,1 % en poids de carbone.

2. Procédé selon revendication 1,
**caractérisé en ce que**
l'alliage cobalt-chrome contient en plus 0 - 0,2 % en poids au moins d'un des éléments bore, yttrium, 0 - 2 % en poids d'au moins un des éléments vanadium, silicium, cuivre, zinc, niobium, 0 -10 % en poids de gallium, 0 - 5 % en poids de germanium et 0 -1 % en poids d'au moins un des éléments cérium, lanthane.

3. Procédé selon revendication 2,
**caractérisé en ce que**
l'alliage ne contient pas plus que 50 % en poids de chrome, de tungstène et de rhénium.

4. Procédé selon au moins une des revendications précédantes,
**caractérisé en ce que**
la relation chrome : tungstène + rhénium est entre environ 2:3 et environ 2:1, respectivement entre environ 2:3 et environ 3:2.

5. Procédé selon au moins une des revendications précédantes,
**caractérisé en ce que**
la teneur en chrome + tungstène est au moins 30 % en poids et au maximum 50 % en poids, et la relation de chrome : tungstène est entre environ 3:4 et environ 4:3.

6. Procédé selon au moins une des revendications précédantes,
**caractérisé en ce que**
l'alliage contient 50 - 65 % en poids de cobalt, 15 - 22 % en poids de chrome, 15 - 22 % en poids de tungstène, 4 - 11 % en poids de fer et 1 - 3 % en poids d'aluminium.

7. Procédé selon au moins une des revendications précédantes,
**caractérisé en ce que**
la prothèse dentaire fabriquée est recouverte sans trempe précédente.

8. Utilisation d'un alliage cobalt-chrome, constitué de
43 - 68 % en poids de cobalt
12 -30 % en poids de chrome
8 - 25 % en poids de tungstène
0 -13 % en poids de fer
0 - 30 % en poids de manganèse
0 -10 % en poids de molybdène
0 - 5 % en poids d'au moins un des éléments
aluminium, tantale, rhénium, titane et moins que 0,1 % en poids de carbone pour la fabrication d'une prothèse dentaire par procédé fusion laser et/ou par procédé de frittage.

9. Utilisation d'un alliage cobalt-chrome selon revendication 8,
qui contient en plus
0 - 0,2 % en poids d'au moins un des éléments bore, yttrium, 0 - 2 % en poids d'au moins un des éléments vanadium, silicium, cuivre, zinc, niobium, 0 -10 % en poids de gallium, 0 - 5 % en poids de germanium et 0 -1 % en poids d'au moins un des éléments cérium, lanthane.

10. Utilisation de l'alliage cobalt-chrome selon revendication 9,
qui ne contient pas plus que 50 % en poids de chrome, tungstène et rhénium.

11. Utilisation de l'alliage cobalt-chrome selon au moins une des revendications 8 - 10,
ayant une relation de chrome : tungstène + rhenium entre environ 2:3 et environ 2:1, respectivement entre environ 2:3 et environ 3:2.

12. Utilisation de l'alliage cobalt-chrome selon au moins une des revendications 8-11,
dans lequel la teneur en chrome + tungstène est d'au moins 30 % en poids et au maximum 50 % en poids, et la relation de chrome : tungstène est entre environ 3:4 et environ 4:3.

13. Utilisation de l'alliage cobalt-chrome selon au moins une des revendications 8-12
ayant 50 - 65 % en poids de cobalt, 15 - 22 % en poids de chrome, 15 - 22 % en poids de tungstène, 3 -11 % en poids de fer et 1 - 3 % en poids d'aluminium.

14. Une prothèse dentaire sans revêtement fabriquée d'un alliage cobalt-chrome d'une dureté HV (HV10) ≤ 350 selon au moins une des revendications précédentes par procédé fusion laser et/ou par procédé de frittage.
